# EUROPEAN PATENT APPLICATION

(11) **EP 1 462 135 A1**
(43) Date of publication of application: **29.09.2004**
(21) Application number: 04007047.6
(22) Date of filing: 24.03.2004
(51) Int. Cl.: A61M 5/32

(54) **Shieldable unit dose medical needle assemblies**

(30) Priority: 28.03.2003 US 402904
(71) Applicant: Becton Dickinson and Company, Franklin Lakes, New Jersey 07417-1880 (US)
(72) Inventor: Prais, Alfred W., Hewitt New Jersey 07421 (US); Caizza, Richard James, Vermon New Jersey 07462 (US); Henniger, Gary, Wayne New Jersey 07470 (US); Kateraas, Espen D., Tuxedo NY 10987 (US)
(74) Representative: von Kreisler, Alek, Dipl.-Chem.

(57) **Abstract**

A shieldable unit dose assembly includes a needle holding member, a safety shield member disposed around the needle holding member, and a unit dose needle including a hub which is connected to the needle holding member. The needle holder member includes a male tapering surface and the hub of the unit dose needle includes a female tapering surface for connection therebetween. The unit dose needle includes a solid elongated unit dose needle having a patient end for containing and administering a unit dose of a vaccine. The telescoping safety shield member is disposed around the needle holding member, and is axially movable from a non-shielding position surrounding at least a portion of the needle holding member to a telescoped shielding position encompassing the patient end of the unit dose needle. The safety shield member may be locked into the shielding position.

## Description

### CROSS REFERENCE TO RELATED APPLICATIONS

This application is a continuation-in-part of United States Patent Application Serial No. 10/141,538, filed May 9, 2002, entitled "Medical Needle Assemblies, which claims priority to United States Provisional Patent Application Serial No. 60/344,304, filed December 28, 2001, entitled "Bifurcated Needle Assembly".

### BACKGROUND OF THE INVENTION

### 1. Field of the Invention

The present invention relates to needles for use in medical procedures and, in particular, to safety shielded needles and needle assemblies used in medical procedures.

### 2. Description of Related Art

Bifurcated or forked end needles are well-known for providing a simple and effective means for a doctor to administer a vaccine. During use, the bifurcated tip of the bifurcated needle is put into contact with either a dried or liquid-substance, which adheres to the bifurcated needle tip. The bifurcated needle tip is then put into contact with the skin of the patient who is being administered the vaccination. The skin is either scratched or pierced with the needle tip so that the vaccination material may be absorbed into the skin of the patient. An alternative method of delivering the vaccination includes placing a drop of the vaccine onto the skin of the patient and contacting the skin of the patient with the bifurcated needle tip through the drop of vaccination. Alternatively, a standard pointed needle tip may also be used when the drop of vaccination is applied directly to the skin of the patient.

The bifurcated needle is considered a significant medical advancement because it has allowed more people to be vaccinated with less serum. This has been especially important for those living in less developed areas because of the efficient and easy to use design, as well as the ease of replication.

Vaccination effectiveness, however, is reduced if the bifurcated needle is reused too many times. Moreover, reuse of such vaccination needles exposes patients to the risk of transmission of infectious diseases through percutaneous contact through the skin. Additionally, medical care workers using traditional vaccination needles are at an increased risk of exposure to infectious diseases due to the design of such needles, which makes them difficult to handle, as well as due to the repeated use of such needles.

In particular, bifurcated needles used to administer vaccinations are not traditionally sterilized or packaged in a single-use container that would enable convenient storage and subsequent use. Additionally, such needles have traditionally been difficult to handle in that they typically do not include a hub attached to the opposite end of a needle from the tip, and do not typically include any sort of shield for protection from the needle prior to and during use.

For example, U.S. Patent No. 3,194,237 to Rubin discloses a vaccinating needle having a main shank with a pair of prongs at one end that define a slot of predetermined length, width and depth therebetween to hold an amount of liquid by capillary action. The shank of the needle is of sufficient length so that the non-prong end will function as a handle. U.S. Patent No. 3,948,261 to Steiner discloses a reusable unit dose container for vaccines contained within a rigid receptacle, with a compressible closure for supporting a bifurcated needle bearing dried vaccine. The closure is adapted to support the needle in the container during a lyophilizing process while liquid vaccine is dried on the needle. The closure has grooves which permit the vaporized liquid from the vaccine to be withdrawn from the receptacle during lyophilizing, and can further seal the container.

While shieldable syringes or needle assemblies are well known in the art for needles used to inject fluids and medicine into the circulatory system of the patient (i.e., veni-puncture), such shielding has not previously been used in connection with vaccination needles such as bifurcated needles. Thus, a need exists for a safety assembly for use with a unit dose vaccination needle that is easily manufactured, simple to use, and may be shielded to prevent accidental needle stick wounds.

### SUMMARY OF THE INVENTION

The present invention is directed to a shieldable unit dose assembly for administering a unit dose of vaccine. The assembly generally includes a needle holding member having a proximal end and a distal end, with the distal end including a male tapering surface. The assembly further includes a unit dose needle assembly supported at the distal end of the needle holding member. The unit dose needle assembly includes a solid elongated unit dose needle having a patient end for containing and administering a unit dose of a vaccine and a hub supporting the unit dose needle. Desirably, the unit dose needle is a bifurcated needle having two pointed prongs capable of penetrating or abrading the skin of the patient, with the prongs being separated by a U-shaped channel capable of holding a unit dose of vaccine. The hub includes a female tapering surface in engagement with the male tapering surface at the distal end of the needle holding member. The assembly further includes a telescoping safety shield member disposed around the needle holding member and axially movable from a non-shielding position surrounding at least a portion of the needle holding member to a telescoped shielding position encompassing the patient end of the unit dose needle.

The needle holding member may include a locking member at a distal end thereof, and the safety shield member may include an internal locking recess at a proximal end thereof adapted to cooperate with the locking member when the safety shield member is in the shielding position to prevent the safety shield member from returning to the non-shielding position. The locking member may comprise a locking collar having an exterior wedge for engaging the internal locking recess to prevent the safety shield member from returning to the non-shielding position. Further, the assembly may include means for indicating when the safety shield member is in the fully shielding position, such as a color indicator.

In a further embodiment, the present invention is directed to a shieldable needle assembly including a unit dose needle assembly connected to a needle holder assembly. The unit dose needle assembly includes a hub having a female tapering surface at a proximal end thereof and a solid elongated unit dose needle extending from a distal end thereof. The unit dose needle is of a length which is capable of retrieving a unit dose of a vaccine from a separate container, and includes a patient end for containing and administering the unit dose of a vaccine, such as a bifurcated pronged end with a U-shaped channel. The needle holding assembly includes an elongated body extending between a proximal end and a distal end. The distal end includes a male tapering surface in engagement with the female tapering surface of the hub of the unit dose needle assembly, and also includes an annular collar having internal threads in threaded engagement with corresponding structure on the proximal end of the hub. The needle holding assembly further includes a telescoping safety shield member disposed concentrically around the elongated body with at least a portion of the proximal end of the body extending from a proximal end of the safety shield member. The safety shield member is axially movable from a non-shielding position in which the unit dose needle is exposed to a telescoped shielding position encompassing the length of the unit dose needle including the patient end.

In a further embodiment, the present invention is directed to a method for administering a vaccine. In the method, a shieldable medical needle assembly comprising a unit dose needle assembly attached to a needle holding assembly is provided. The unit dose needle assembly includes a solid elongated unit dose needle including a bifurcated pronged patient end for containing a unit dose of a vaccine. The needle holding assembly includes an elongated body with a proximal end and a distal end, and further includes a telescoping safety shield member disposed concentrically around the elongated body with at least a portion of the proximal end of the body extending from the safety shield member. The bifurcated pronged patient end of the unit dose needle is first contacted with a vaccine so as to contain at least a portion of the vaccine on the bifurcated pronged patient end. The bifurcated pronged patient end of the unit dose needle is thereafter contacted with a patient's skin so as to cause the bifurcated pronged patient end to abrade the patient's skin. The safety shield member is thereafter axially moved in a direction toward the bifurcated pronged patient end. As such, the shield member is moved from a non-shielding position in which the safety shield member is disposed concentrically around the elongated body and the unit dose needle is exposed to a telescoped shielding position encompassing the length of the unit dose needle including the bifurcated pronged patient end.

Further details and advantages of the present invention will become apparent from the following detailed description, when read in conjunction with the drawings.

### BRIEF DESCRIPTION OF THE DRAWINGS

FIG. 1 is a perspective view of a shieldable needle assembly of the present invention;

FIG. 2 is an exploded view of the shieldable needle assembly shown in FIG. 1;

FIG. 3 is an exploded perspective view of the unit dose needle assembly of FIG. 1;

FIG. 4 is a side cross-sectional view of the unit dose needle assembly of FIG. 3;

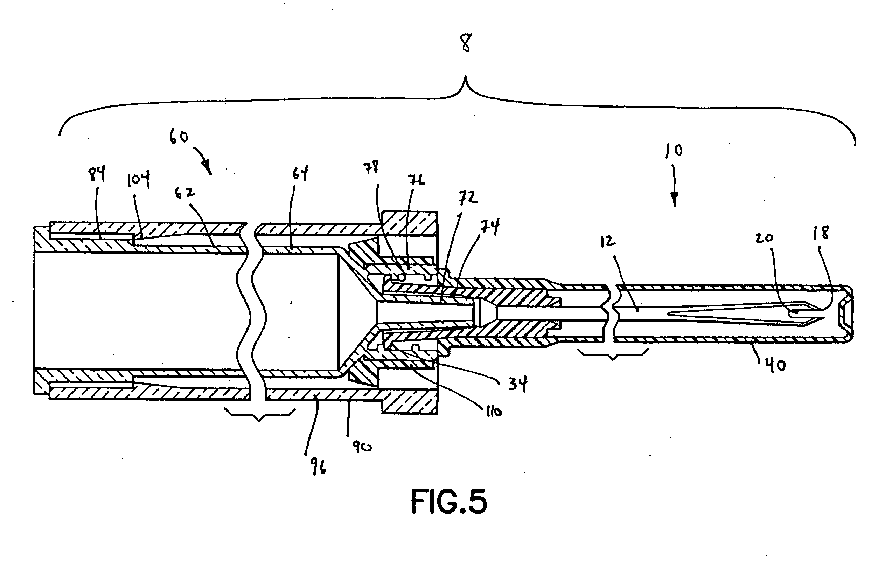

FIG. 5 is a side cross-sectional view of the shieldable needle assembly of FIG. 1;

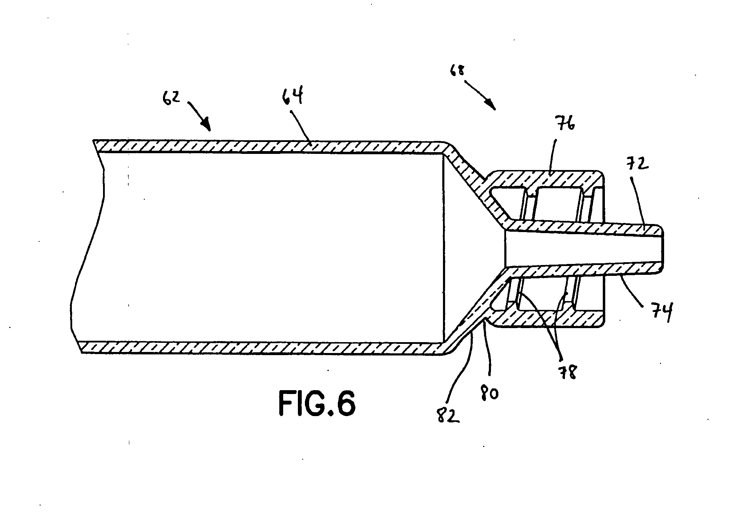

FIG. 6 is an enlarged cross-sectional view of a proximal end of the needle holding member;

FIG. 7 is an enlarged plan view of a locking member adapted for connection to the needle holding member of the present invention;

FIG. 8 is a cross-sectional view of the locking member of FIG. 7;

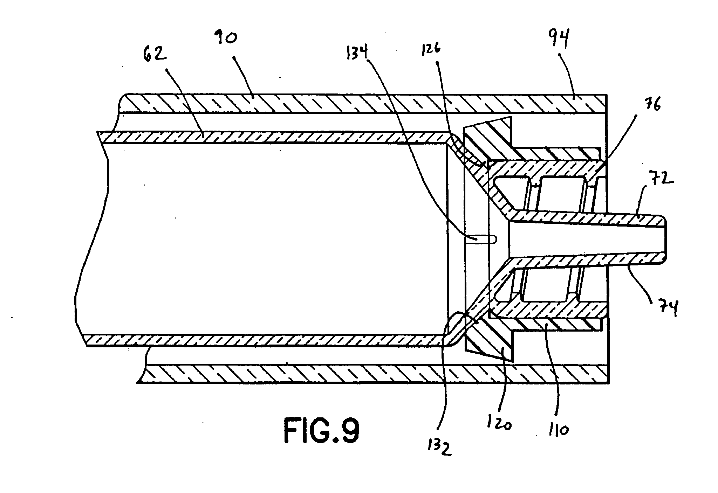

FIG. 9 is an enlarged partial cross-sectional view of the proximal end of the needle holding assembly;

FIG. 10 is a perspective view of the shieldable needle assembly showing the safety shield member in a telescoped, shielding position;

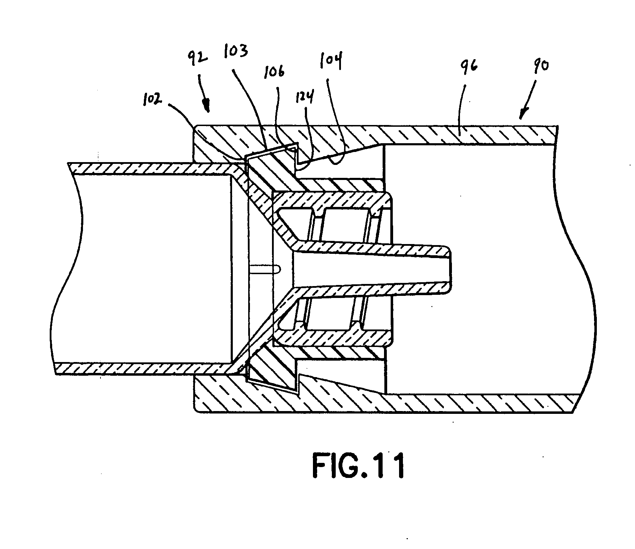

FIG. 11 is a side cross-sectional view showing the connection between the safety shield member, the locking member, and the needle holding member, with the safety shield member in the shielding position as shown in FIG. 10; and

FIG. 12 is a perspective view of a shieldable needle assembly in an alternate embodiment including color indicator means.

### DESCRIPTION OF THE PREFERRED EMBODIMENTS

In the following description and accompanying drawings, like reference numbers, as used in the various figures, refer to like features or elements. The terms top and bottom, as used herein, refer to the orientation of a given element as shown in the drawings.

For purposes of the description hereinafter, the terms "upper", "lower", "right", "left", "vertical", "horizontal", "top", "bottom", and derivatives thereof shall relate to the invention, as it is oriented in the drawing figures. However, it is to be understood that the invention may assume various alternative variations and step sequences except where expressly specified to the contrary. It is also to be understood that the specific devices and processes illustrated in the attached drawings and described in the following text are simply exemplary embodiments of the invention. Hence, specific dimensions and other physical characteristics related to the embodiments disclosed hereinafter are not to be considered limiting.

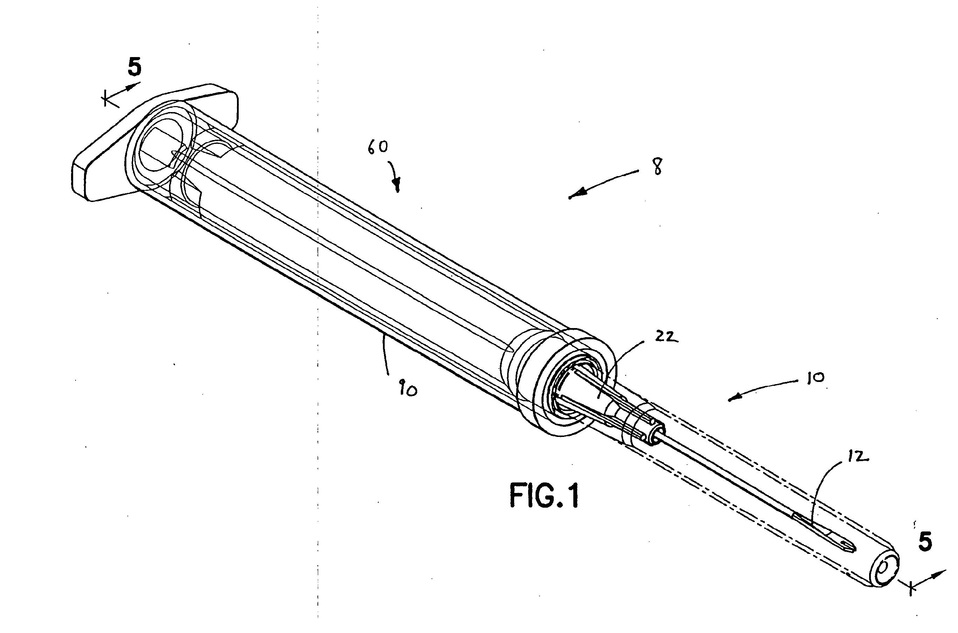

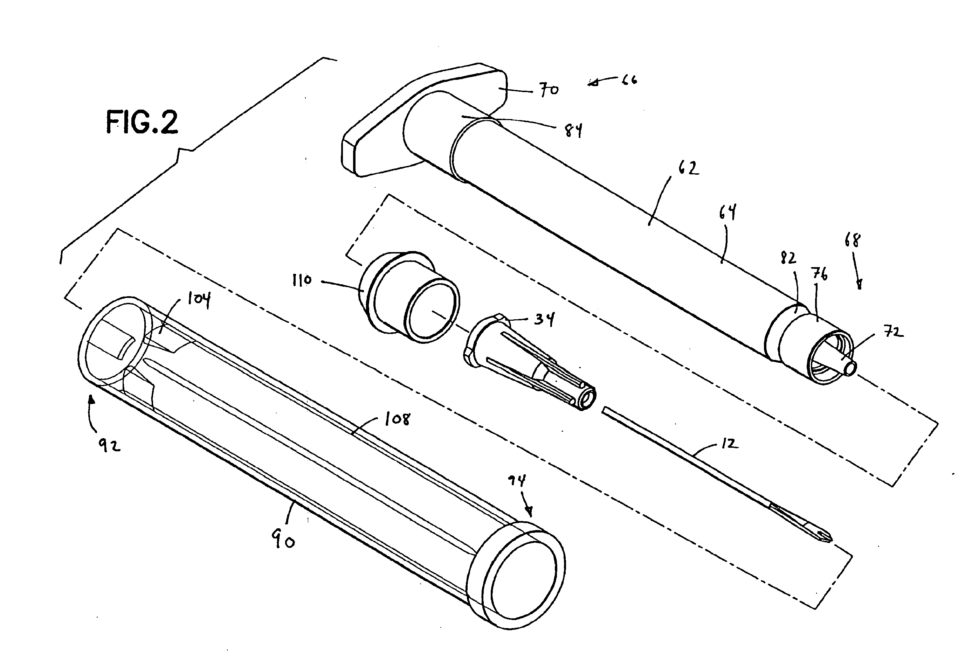

Referring to the drawings in which like reference characters refer to like parts throughout the several views thereof, FIGS. 1-2 depict a shieldable medical needle assembly **8** in accordance with the present invention and the related features. The shieldable assembly **8** includes a unit dose needle assembly **10** connected to a needle holding assembly **60**. The shieldable assembly **8** is intended for use for the administration of vaccines applied to or through the skin of the patient, and is intended as a single-use vaccination needle assembly including features to provide ease of use for the medical practitioner and safety shielding of the needle after use, as will be described in more detail herein.

Generally, needle holding assembly **60** includes needle holding member **62** which is defined by a generally cylindrical body **64** extending between proximal end **66** and distal end **68.** The cylindrical body **64** may be a hollow member, such as a conventional syringe barrel, or may be a solid member. As will be discussed further herein, proximal end **66** of the body **64** of needle holding member **62** desirably includes structure for grasping needle holding member **62,** such as a circumferential flange or a pair of flange tabs **70.**

The needle holding member **62** includes a tapered tip **72** projecting distally therefrom at distal end **68**. Tapered tip **72** includes a male tapering surface such as a male luer taper **74.** It is noted, however, that tapered tip **72** need not have any opening therethrough, and may be a solid member, which may provide additional structural integrity to the tapered tip **72.** Needle holding member **62** may also include a luer collar **76** at distal end **68**, which is generally adjacent tapered tip **72** and generally surrounds tapered tip **72.** Luer collar **76** may include a plurality of internal threads **78** for threadably receiving a hub of a unit dose needle assembly, as explained further herein. The luer collar **76** defines an external groove **80** adjacent an annular shoulder **82** defined by body **64** of needle holding member **62.**

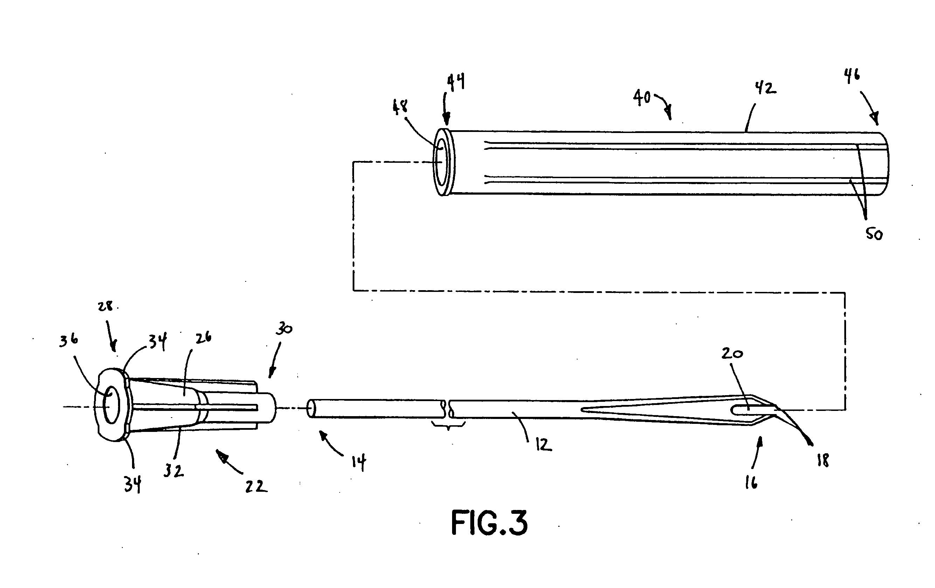

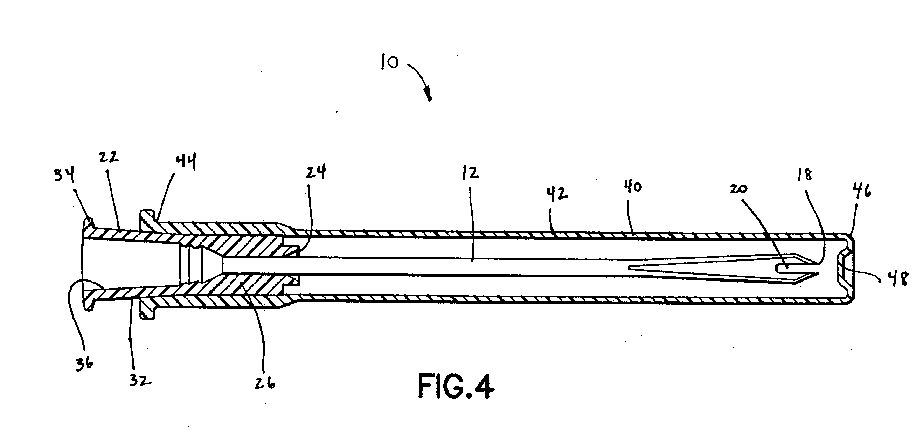

Referring generally to FIGS. 3-4, the shieldable needle assembly **8** further comprises a unit dose needle assembly **10** supported by the needle holding assembly **60** at the distal end **68** of needle holding member **62.** The unit dose needle assembly **10** generally includes a unit dose needle for administering a unit dose of a vaccine. Desirably, the unit dose needle is a bifurcated needle, such as bifurcated needle **12,** which is supported by a hub **22**, and enclosed within a needle shield **40**. While unit dose needle assembly **10** is described herein in terms of a preferred embodiment including bifurcated needle **12,** unit dose needle assembly **10** may include any unit dose needle capable of administering a unit dose of a vaccine, such as in a dry powder or liquid form, as is well known in the art. Desirably, the unit dose needle is sterilized and maintained in a sterile condition until used, and may be safely-disposed of along with the shieldable medical assembly 8 at the conclusion of a vaccination procedure, for example.

Bifurcated needle **12** is a solid, elongated needle structure extending between a non-patient end at proximal end **14,** and an opposed prong end or patient end at distal end **16**. Bifurcated needle **12** is provided with two sharp prongs **18** positioned at a distal end **16** of the needle. The prongs **18** are separated by a U-shaped channel **20** configured to hold a unit dose of vaccine. The prongs **18** are intended to penetrate or abrade the skin of the patient to administer the vaccine disposed in the U-shaped channel **20**. Bifurcated needle **12** may be constructed of any material known in the art, such as metal or plastic, and is desirably constructed of a medical grade surgical steel.

Hub **22** is fixedly attached to the proximal end **14** of bifurcated needle **12,** such as through an adhesive joint **24.** Adhesive joint **24** may be provided through any adhesive capable of fixedly attaching or adhering bifurcated needle **12** to hub **22**, such as an epoxy or equivalent adhesive. Hub **22** includes a hub housing **26** including a proximal end **28** and a distal end **30,** with at least a portion of the external surface of hub housing **26** defining an outer tapered surface **32** extending therealong. Distal end **30** of hub **22** may include an internal bore having an internal diameter of approximately the same size as the outer diameter of the proximal end **14** of bifurcated needle **12**, for accommodating and fixedly adhering bifurcated needle **12** within such an internal bore of hub **22.**

The hub **22** further includes means for attaching the unit dose needle assembly **10** to the needle holding assembly **60**. Desirably, the hub includes luer lugs **34** extending about the proximal end thereof, with an internal luer taper **36** which extends internally within a portion of hub housing **26**. Internal luer taper **36** is a female tapering surface which extends internally within at least a portion of hub housing **26**, for engagement with the male luer taper **24** of needle holding member **62.** In this manner, unit dose needle assembly **10** can be attached to needle holding assembly **60,** which can provide shieldable medical assembly **8** with an appropriate handle portion for the assembly, thereby facilitating ease of use of the assembly. While not shown, internal luer taper **36** may include internal threads for establishing threaded engagement with a corresponding threaded surface of the needle holding member **62**.

While the present invention is described in terms of a unit dose needle assembly **10** separately attached to a needle holding assembly **60**, it is contemplated that the unit dose needle assembly **10** and the needle holding assembly **60** can be permanently attached or integrally formed, thereby providing shieldable medical assembly **8** as a single structure.

Unit dose needle assembly **10** may further include a needle shield **40** extending about bifurcated needle **12.** Needle shield **40** is of a generally tubular hollow construction, including tubular shield housing **42** extending between a proximal end **44** and a distal end **46**, with the tubular shape of shield housing **42** forming an internal opening **48** extending through needle shield **40.** Proximal end **44** of needle shield **40** is generally open-ended, forming a passage for access to internal opening **48,** while distal end **46** is closed-ended, forming a wall. Needle shield **40** extends about bifurcated needle **12,** thereby containing bifurcated needle **12** within internal opening **48.** Proximal end **44** may further be provided with a lip extending circumferentially about the open ended passage. Proximal end **44** removably engages the hub **22** along the tapered surface **32** to form an air-tight seal, completely concealing the bifurcated needle **12** and associated prongs **18** therein in a sterile, air-tight manner.

The needle shield **40** serves to protect the bifurcated needle **12** from damage and exposure to soils or other contaminants during shipping and storage, and prior to use of the unit dose needle assembly. The needle shield **40** also provides protection to personnel from needle sticks prior to removing the needle shield **40** for use.

The hub **22** and needle shield **40** may be constructed of any material, and are desirably constructed of a moldable plastic material. Suitable moldable plastics include, but are not limited to polyethylenes, polypropylenes, polyamides, polyesters and fluorinated polyethylenes. Preferably, hub **22** is constructed of a rigid material, while needle shield **40** may be formed from a non-rigid material. By non-rigid material, what is meant is a material that is sufficiently flexible to conform to the tapered surface **32** of the hub **22** to form an air-tight seal. While needle shield **40** may be desirably formed from polypropylene, any material known by those of skill in the art to facilitate sterilization of the unit dose needle **10** may also be used. Needle shield **40** may further include external ribs **50** integrally molded with shield housing **42** and extending longitudinally along the outer surface of shield housing **42** between proximal end **44** and distal end **46.** Such external ribs **50** provide further structural integrity to needle shield **40,** which is particularly useful during packaging and storage.

The unit dose needle, i.e., bifurcated needle **12,** may be used to administer vaccine that is in any suitable form. Suitable physical forms for the vaccine include, but are not limited to liquids, such as solutions, emulsions, and dispersions, or dry powders. Typically, the vaccine will be in a liquid form. Moreover, the vaccine may be associated with unit dose needle assembly **10** during storage, and may therefore be contained within the U-shaped channel **20** prior to removal of needle shield **40.** Alternatively, the vaccine may be provided as a separate component in a separate container, with bifurcated needle **12** being contacted with the vaccine after removing needle shield **40** therefrom just prior to use of the needle for administration of the vaccine. In this manner, it is important that the unit dose needle be of sufficient length so as to be able to contact the vaccine within its storage container. For example, vaccines are typically stored in closed end containers or vials. The unit dose needle must be of a sufficient length so as to be able to extend into the container or vial and contact the vaccine therein. Typically, the bifurcated needle **12** will have a length of about 1.8 inches, and the unit dose needle including the hub **22** will have an overall length of about 2.2 inches.

As noted, needle shield **40** sealingly mates with hub **22** to provide an air-tight connection therebetween, with bifurcated needle **12** contained within the air-tight environment in internal opening **48** of needle shield **40.** Such an air-tight arrangement provides unit dose needle assembly **10** as a self-contained assembly, in the form of a complete, shielded, sterile, single-use unit dose needle assembly, which can be shipped in this form. Alternatively, this unit dose needle assembly **10** may be further packaged to provide additional sterility to the assembly.

As noted, unit dose needle assembly **10** is connected with needle holding assembly **60**. Needle holding assembly **60** further includes a telescoping safety shield member **90** disposed around the body **64** of needle holding member **62.** The safety shield member **90** includes a proximal end **92**, a distal end **94,** and a rigid tubular sidewall **96** extending therebetween. The overall length of safety shield member **90** is slightly less than the body **64** of needle holding member **62**, thereby providing a portion of needle holding member **62** to grasp for relative axial movement of the safety shield member **90** and the needle holding member **62.** The overall length of safety shield member **90** is also sufficient so as to completely encompass the unit dose needle, i.e. bifurcated needle **12** and prongs **18.**

Interior portions of the safety shield member **90** adjacent the proximal end **92** include an inward directed locking shoulder **98** having a proximal face **100** and a radially aligned distal locking face **102.** A locking recess **103** is defined distally forward of the locking shoulder **98.** The locking recess **103** is formed by a locking face of the locking shoulder **98** and a locking pawl **104** located distally forward of the locking shoulder **98.** The locking pawl **104** includes a locking pawl face **106,** which further defines the locking recess **103.** The interior surface of safety shield member **90** may further include longitudinal ribs extending therealong, which facilitate axial movement of safety shield member **90** during use, as will be explained in further detail herein.

Needle holding assembly **60** further includes a locking member in the form of locking collar **110** disposed around needle holding member **62** at distal end **68** thereof. The locking collar **110** includes a proximal end **114**, a distal end **116**, and a generally tubular sidewall **118** extending therebetween. The locking collar **110** also includes a portion cooperating with the external groove **80**. The locking collar **110** is adapted to connect and lock the telescoping safety shield member **90** to the needle holding member **62**.

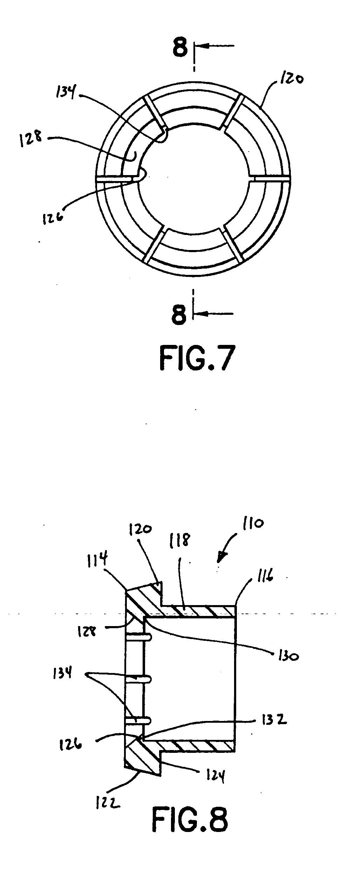

More particularly, the locking collar **110** includes an exterior wedge **120** having a frustom-shaped proximal face **122** and a radially aligned distal face **124.** The locking collar **110** further includes an interior wedge **126** having an outward tapered proximal face **128** and a frustom-shaped distal face **130**. The proximal and distal faces of the interior wedge **126** form a barbed portion **132** adapted to cooperate with the external groove **80** defined in the body **64** of needle holding member **62.** As shown in FIGS. 7-8, the locking collar **110** is further defined by a plurality of slots **134** extending distally from the proximal end **114.** The slots **134** permit the locking collar **110** to tightly engage the external groove **80** to secure the connection between the safety shield member **90** and the needle holding member **62** as discussed hereinafter.

The needle holding member **62,** shield member **90,** and the locking member **120** may be made of any of the materials identified previously in connection with the unit dose needle assembly **10** and, more particularly, the hub **22** of the unit dose needle assembly **10.** Desirably, the locking collar **110** is made of a sufficiently resiliently deformable material such that it may be positioned over the distal end **68** of the body **64** and resiliently engage the external groove **80** defined in the needle holding member **62**.

The shieldable medical assembly **8** may be assembled by initially mounting the safety shield member **90** over the needle holding member **62.** The outside diameter of body **64** of the needle holding member **62** adjacent proximal end **66** may be formed slightly larger than the outside diameter of the remainder of body **64** forming an enlarged portion **84,** so that the proximal end **66** of the needle holding member **62** may frictionally and releasably engage locking shoulder **98** for holding the safety shield member **90** in the proximal or non-shielding position on the needle holding member **62.** The locking collar **110** may then be advanced on to the distal end **68** of the needle holding member **62,** with the slots **134** defined in the locking collar **110** permitting the locking collar **110** to expand and fit onto the tip **72**. In particular, the locking collar **110** engages the luer collar **76** located at the distal end **68** of the needle holding member **62.** The interior wedge **126** of the locking collar **110** engages the external groove **80** defined in the luer collar **76.** The tapered face **128** of the locking collar **110** aids in the expansion of the locking collar **110** as it is positioned over the luer collar **76**. Once the locking collar **110** engages the external groove **80,** the locking collar **110** returns to an undeflected condition, with the interior wedge **126** securely engaged in the external groove **80.**

Once the safety shield member **90** is positioned about the needle holding member **62** and locking collar **110,** the unit dose needle assembly **10** may be connected to the needle holding member **62.** In particular, the hub **22** of the unit dose needle assembly **10** is threadably engaged with internal threads **78** defined in the luer collar **76**, with female internal luer taper **36** frictionally engaging male external luer taper **74.** The shieldable medical assembly **8** is now ready for use in a vaccination procedure, as an example.

The unit dose needle may be pre-supplied with a vaccine liquid. In which case, once the needle shield is removed, the unit dose needle is ready to be used in the vaccination procedure. Alternatively, the needle shield may be removed and the unit dose needle may be contacted with a vaccine. Once used in a vaccination procedure, the unit dose needle is considered contaminated. To prevent accidental needle sticks with the used unit dose needle and to prevent unauthorized use of the shieldable medical assembly, the technician using the assembly urges the safety shield member **90** distally forward relative to the needle holding member by holding flange tabs **70** and forcing the safety shield member **90** toward the distal end **68**. Initial distal forces on the safety shield member **90** will disengage the locking shoulder **98** from its frictional engagement with the slightly enlarged portion **84** at the proximal end **66** of the needle holding member **62.** Further distal movement will proceed without substantial interference.

Moreover, interior ribs **108** on the interior surface of tubular wall **96** of safety shield member **90** assist in proper axial movement of safety shield member **90** with respect to needle holding member **62.** In particular, interior ribs **108** glide along an outside edge of exterior wedge **120** of locking collar **110** in a frictional manner during such movement from the non-shielding position to the shielding position. Such frictional gliding prevents safety shield assembly **90** from being easily rotated about needle holding member **62**, and therefore assists in maintaining axial movement thereof.

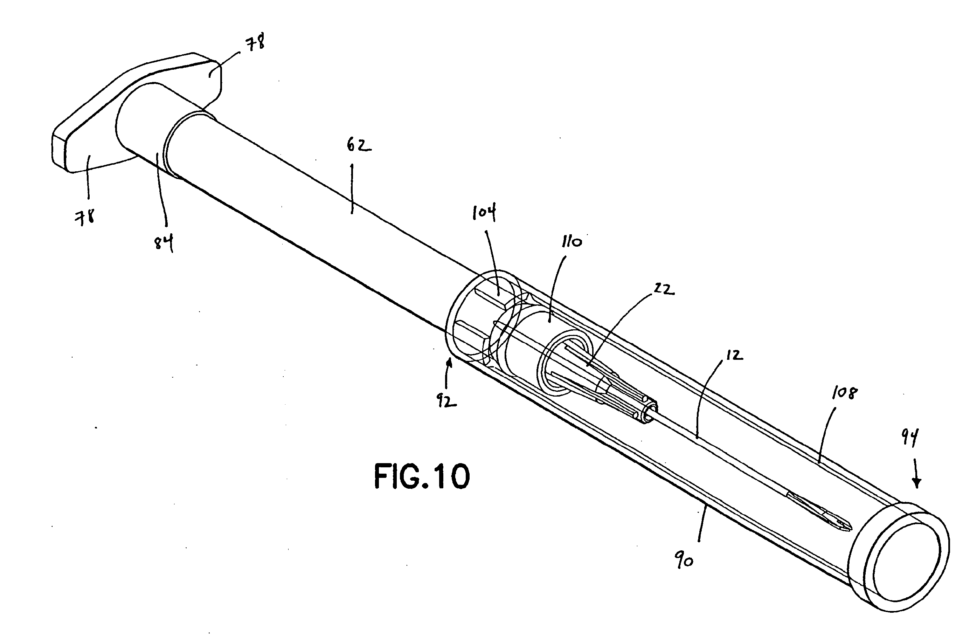

As the safety shield member **90** is telescoped over the unit dose needle toward a shielding position-as shown in FIG. **10,** the proximal-pawl face **106** of the locking pawl **104** engages the exterior wedge **120** on the locking collar **110.** This initial engagement will generate a slight radial expansion of the safety shield member **90** near its proximal end **92** to prevent further distal movement of the locking shoulder member **98.** After further distal movement of the locking shield member **90,** the exterior wedge **120** is received in a locking recess **103** between the distal locking face **102** and the pawl face **106,** and the safety shield member resiliently returns to an unexpanded condition. The engagement of the exterior wedge **120** in the locking recess **103** prevents distal or proximal movement of the safety shield member **90.** Additionally, the engagement of the exterior wedge **120** and the locking recess **103** simultaneously tightens the interior wedge **126** into the external groove **80** in the luer collar **76.** Further, additional distal movement is also prevented by engagement of the locking shoulder **98** with the proximal face **122** of the exterior wedge **120**. In summary, using normal fingertip force, movement of the safety shield member **90** is prevented by the interior and exterior wedges **126, 120,** respectively, on the locking collar **110,** which mate with the external groove **80** in the luer collar **76** and the locking recess **103** in the safety shield member **90.** The above described wedging interaction between the safety shield member **90** and locking collar **110** are typically sufficient to prevent tampering with the safety shield member **90** once extended to its shielding position.

As indicated previously, a return or proximal movement of the safety shield member 90 to the non-shielding position with the unit dose needle exposed is prevented by the interaction between the proximal face of the locking pall on the distal face of the exterior wedge on the locking collar. Any proximally directed forces will merely urge the proximal face of the exterior wedge against the tapered shoulder of the needle holding member located adjacent the luer collar. Thus, the re-exposure of the used unit dose needle is positively prevented.

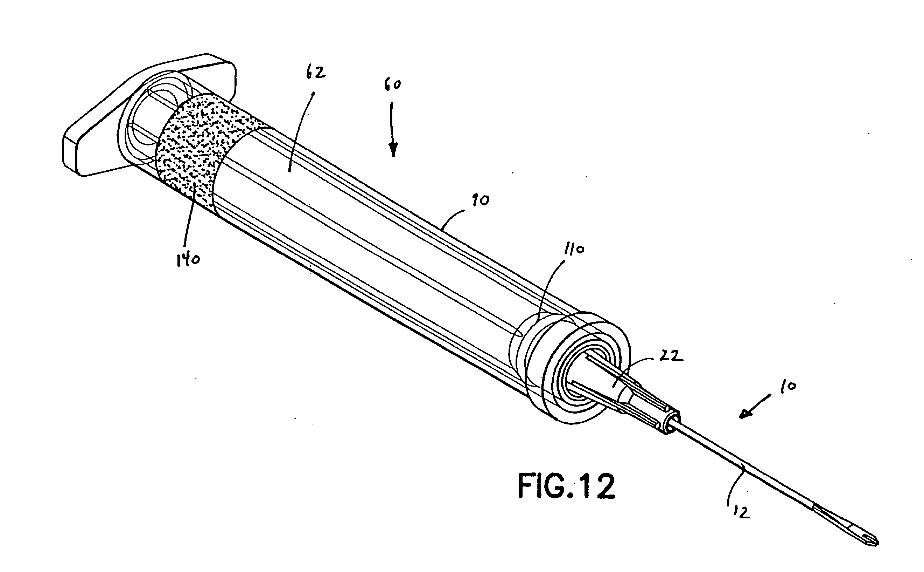

It is further contemplated that the shieldable medical assembly **8** may include means for indicating that the safety shield member **90** is in the shielding position, or more desirably, locked in place in the fully extended position shielding the unit dose needle. Such means may include a visual indicator, a tactile indicator, an audible indicator, or the like. For example, as shown in FIG. 12, corresponding visual structure may be present on the safety shield member **90** an the locking collar **110** to confirm proper engagement therebetween. In particular, safety shield member **90** may include a circumferential color band such as green band **140** at the proximal end **92** thereof. Locking collar **110** may further include a color band or may be constructed of a material which is colored in a complementary color of the color band on the safety shield member **90,** such as a red color. In this manner, when safety shield member **90** is extended to the shielding position, the green band **140** will overlap the red locking collar **110,** thereby creating a black shaded color portion. This change in color provides a visual indication that the shieldable medical assembly **8** is in the shielding position and can be appropriately discarded. Also, the interaction between the locking shoulder **98** and the corresponding surfaces of the locking collar **110** create a tactile and or audible indication that the safety shield member **90** has been moved to the shielding position and locked in place.

The present invention provides an effective method for use of a unit dose medical needle by providing a unit dose needle with a proper handle portion and an appropriate shielding mechanism. In particular, the needle holding member **62** provides an effective handle portion for using the bifurcated needle **12** to administer a vaccine. Also, the overall dimensions of the safety shield member **90** provide an effective shield portion for the entire length of bifurcated needle **12** after use thereof.

It is important to note that as the unit dose needle is merely intended to deliver a unit dose of a vaccine which is present on the tip thereof, it is of solid construction. Accordingly, there is need for any internal fluid communication between the unit dose needle assembly **10** and the needle holding assembly 60. The needle holding assembly **60** is merely provided to act as a structure which is capable of providing an effective handle for the unit dose needle, and to provide for effective safety shielding of the unit dose needle after use thereof.

While the present application is satisfied by embodiments in may different forms, there is shown in the drawings and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope and spirit of the invention. The scope of the invention will be measured by the appended claims and their equivalents.

## Claims

1. A shieldable unit dose assembly, comprising:
a needle holding member having a proximal end and a distal end, the distal end including a male tapering surface;
a unit dose needle assembly supported at the distal end of the needle holding member, the unit dose needle assembly comprising a solid elongated unit dose needle having a patient end for containing and administering a unit dose of a vaccine and a hub supporting the unit dose needle, the hub including a female tapering surface in engagement with the male tapering surface at the distal end of the needle holding member; and
a telescoping safety shield member disposed around the needle holding member and axially movable from a non-shielding position surrounding at least a portion of the needle holding member to a telescoped shielding position encompassing the patient end of the unit dose needle.

2. The shieldable unit dose assembly of claim 1, further comprising structure for preventing the safety shield member from returning to the non-shielding position.

3. The shieldable unit dose assembly of claim 2, wherein the needle holding member includes an external groove proximate the distal end with a locking member disposed around the needle holding member and including a portion cooperating with the external groove, and wherein the safety shield member includes an internal locking recess at a proximal end thereof adapted to cooperate with the locking member when the safety shield member is in the shielding position to prevent the safety shield member from returning to the non-shielding position.

4. The shieldable unit dose assembly of claim 3, wherein the locking member comprises a locking collar having an exterior wedge for engaging the internal locking recess to prevent the safety shield member from returning to the proximal position.

5. The shieldable unit dose assembly of claim 3, wherein the locking member comprises a locking collar having an interior wedge for engaging the external groove defined in the needle holding member.

6. The shieldable unit dose assembly of claim 3, wherein the locking member is tubular shaped and made of a resiliently deformable material such that the locking member may be positioned over the distal end of the needle holding member and resiliently engage the external groove defined in the needle holding member.

7. The shieldable unit dose assembly of claim 1, wherein the unit dose needle comprises a bifurcated needle having two pointed prongs capable of penetrating or abrading the skin of a patient, the prongs separated by a U-shaped channel capable of holding a unit dose of vaccine.

8. The shieldable unit dose assembly of claim 1, further comprising means for indicating that the safety shield member is in the shielding position.

9. The shieldable unit dose assembly of claim 1, wherein the distal end of the needle holding member includes an annular collar adjacent the male tapering surface including internal threads, and wherein the hub includes structure for threaded engagement with the internal threads of the annular collar when the female tapering surface is in engagement with the male tapering surface.

10. The shieldable unit dose assembly of claim 1, further comprising structure for maintaining the safety shield member in the non-shielding position prior to movement to the shielded position.

11. The shieldable unit dose assembly of claim 1, wherein the safety shield member includes a plurality of internal ribs extending axially along an internal surface thereof.

12. The shieldable unit dose assembly of claim 1, further comprising a removable cover enclosing the unit dose needle and frictionally engaging the hub.

13. A shieldable unit dose assembly, comprising:
a needle holding member having a proximal end and a distal end, the needle holding member defining an external groove proximate the distal end, the distal end including a male tapering surface and an annular collar including internal threads adjacent the male tapering surface;
a locking member disposed around the needle holding member and including a portion cooperating with the external groove;
a unit dose needle assembly supported at the distal end of the needle holding member, the unit dose needle assembly comprising a solid elongated unit dose needle having a patient end including two pointed prongs capable of penetrating or abrading the skin of a patient and separated by a U-shaped channel capable of holding a unit dose of vaccine, and a hub supporting the unit dose needle, the hub including a female tapering surface in engagement with the male tapering surface at the distal end of the needle holding member and including structure for threadably engaging the internal threads of the annular collar; and
a telescoping safety shield member disposed around a portion of the needle holding member and locking member and axially movable from a non-shielding position encircling the needle holding member to a telescoped shielding position encompassing the patient end of the unit dose needle, the safety shield member defining an internal locking recess at a proximal end thereof adapted to cooperate with the locking member in the distal position of the safety shield member to prevent the safety shield member from returning to the non-shielding position.

14. The shieldable unit dose assembly of claim 13, wherein the locking member comprises a locking collar having an exterior wedge for engaging the internal locking recess to prevent the safety shield member from returning to the non-shielding position.

15. The shieldable unit dose assembly of claim 13 wherein the locking member is tubular shaped and made of a resiliently deformable material such that the locking member may be positioned over the distal end of the needle holding member and resiliently engage the external groove defined in the needle holding member.

16. The shieldable unit dose assembly of claim 13, further comprising a removable safety cap enclosing the unit dose needle and frictionally engaging the hub.

17. A shieldable unit dose assembly, comprising:
a unit dose needle assembly comprising a hub having a female tapering surface at a proximal end thereof and a solid elongated unit dose needle extending from a distal end thereof, the unit dose needle having a length capable of retrieving a unit dose of a vaccine from a separate container and having a patient end for containing and administering the unit dose of a vaccine;
a needle holding assembly having an elongated body with a proximal end and a distal end, the distal end including a male tapering surface in engagement with the female tapering surface of the hub of the unit dose needle assembly and an annular collar having internal threads in threaded engagement with corresponding structure on the proximal end of the hub, the needle holding assembly further including a telescoping safety shield member disposed concentrically around the elongated body with at least a portion of the proximal end of the body extending from the safety shield member, the safety shield member being axially movable from a non-shielding position in which the unit dose needle is exposed to a telescoped shielding position encompassing the length of the unit dose needle including the patient end.

18. The shieldable unit dose assembly of claim 17, wherein the elongated body includes an external groove proximate the distal end thereof, and the needle holding assembly further comprises a locking collar disposed about a portion of the elongated body cooperating with the external groove, and wherein the safety shield member includes an internal locking recess at a proximal end thereof adapted to cooperate with the locking collar when the safety shield member is in the shielding position to prevent the safety shield member from returning to the non-shielding position.

19. The shieldable unit dose assembly of claim 18, wherein the unit dose needle comprises a bifurcated needle having two pointed prongs capable of penetrating or abrading the skin of a patient, the prongs separated by a U-shaped channel capable of holding a unit dose of vaccine.

20. The shieldable unit dose assembly of claim 17, further comprising a removable safety cap enclosing the unit dose needle and frictionally engaging the hub.

21. A method for administering a vaccine comprising:
providing a shieldable medical needle assembly comprising a unit-dose needle assembly attached to a needle holding assembly, the unit dose needle assembly including a solid elongated unit dose needle including a bifurcated pronged patient end for containing a unit dose of a vaccine, the needle holding assembly having an elongated body with a proximal end and a distal end, and further including a telescoping safety shield member disposed concentrically around the elongated body with at least a portion of the proximal end of the body extending from the safety shield member;
contacting the bifurcated pronged patient end of the unit dose needle with a vaccine so as to contain at least a portion of the vaccine on the bifurcated pronged patient end;
contacting the bifurcated pronged patient end of the unit dose needle with a patient's skin so as to cause the bifurcated pronged patient end to abrade the patient's skin; and
axially moving the safety shield member in a direction toward the bifurcated pronged patient end from a non-shielding position in which the safety shield member is disposed concentrically around the elongated body and the unit dose needle is exposed to a telescoped shielding position encompassing the length of the unit dose needle including the bifurcated pronged patient end.
